# EUROPEAN PATENT APPLICATION

(11) **EP 1 548 439 A1**
(43) Date of publication of application: **29.06.2005**
(21) Application number: 03795428.6
(22) Date of filing: 12.09.2003
(51) Int. Cl.: G01N 33/543, G01N 33/547, G01N 33/566

(54) **FIXATION CARRIER AND SOLID PHASE**

(30) Priority: 13.09.2002 JP 2002267636
(71) Applicant: Hitachi Chemical Company, Ltd., Tokyo 163-0449 (JP)
(72) Inventor: NAKAJIMA, Bunichirou, c/o HITACHI CHEM. CO., LTD., Tsukuba-shi, Ibaraki 300-4247 (JP); TAKEDA, Shinji, c/o HITACHI CHEMICAL CO., LTD., Hitachi-shi, Ibaraki 317-0061 (JP); SAWAZAKI, Takeshi, c/o HITACHI CHEMICAL CO., LTD., Hitachi-shi, Ibaraki 317-0061 (JP); MAEKAWA, Baku, c/o HITACHI CHEMICAL CO., LTD., Tokyo 108-0023 (JP); SUTO, Kunihiro, c/o HITACHI CHEMICAL CO., LTD., Tsukuba-shi, Ibaraki 300-4247 (JP)
(74) Representative: HOFFMANN - EITLE
(86) International application number: PCT/JP2003/011710
(87) International publication number: WO 2004/025300

(57) **Abstract**

The object of the present invention is to provide an immobilization support having an optimum support surface for immobilizing various types of materials having different properties with specificity, efficiency, and good reproducibility, and to provide a solid phase having improved characteristics by use of the above support. The immobilization support of the present invention is provided with an electrolyte thin film on the surface of the support. This immobilization support can be applied to the fabrication of a solid phase used in, for example, an antigen-antibody reaction, nucleic acid hybridization, a receptor assay, or a biosensor.

## Description

### Technical Field

The present invention relates to an immobilization support that is used in, for example, an antigen-antibody reaction on a solid phase, nucleic acid hybridization on a solid phase, a receptor-ligand reaction on a solid phase, an enzyme-substrate reaction on a solid phase, or cell immobilization, and to a solid phase employing the support.

### Background Art

Immobilization supports used in systems measuring an antigen-antibody reaction on a solid phase, nucleic acid hybridization on a solid phase, a receptor-ligand reaction on a solid phase, or an enzyme-substrate reaction on a solid phase employ, as a material, a plastic such as polystyrene or polycarbonate, a biopolymer material such as cellulose, a glass, a ceramic resin, a metal, a conductive plastic, etc.; the material is selected in order to control the amount of immobilized substance bound to these supports, and when a suitable support cannot be obtained by selection of the material, the surface is subjected to modification by plasma, gamma rays, ultraviolet rays, ozone, etc. so as to optimize the immobilization support. However, conventional optimization methods have problems in terms of the reproducibility of modification conditions, etc., and it is difficult to uniformly modify the surface of a solid phase such as microplates or latex particles. In particular, it is very difficult to carry out optimum surface modification so as to efficiently bind various types of substances having different properties only by changing some limited conditions. It is therefore necessary to expend a large amount of effort in examining the conditions for immobilizing a target substance on a solid phase, and even then immobilization is totally impossible in some cases. There has therefore been a strong desire for an immobilization support that enables the immobilization of a substance to be freely controlled.

With regard to the above-mentioned background art, a patent publication (International Patent Application WO 97/19978) with the title 'Granular carrier for immobilizing microbial cells and apparatus for producing the granular carrier' discloses a granular carrier for immobilizing microbial cells that is formed by adding dropwise, to an aqueous medium containing an alkali metal ion or a polyvalent metal ion, a liquid composition comprising (a) a hydrophilic photocurable resin having at least two ethylenically unsaturated bonds per molecule, (b) a photopolymerization initiator, and (c) a water-soluble macromolecular polysaccharide having the ability to effect gelling by contact with an alkali metal ion or a polyvalent metal ion, so as to gel the composition into granular, followed by irradiation with actinic radiation so as to cure the granular, the granular carrier for microbial cell immobilization having a specific gravity of 1.00 to 1.20, a contact angle with an n-paraffin on the surface thereof of 2° to 30°, and a surface that a microorganism can suitably attach to. Here, the irradiation with actinic radiation is carried out in order to increase the strength of the granular carrier as a result of photopolymerization of the photocurable resin (p. 14, lines 6-8), and the publication does not disclose further modification of the surface of the granular carrier so obtained.

Furthermore, a patent publication (Japanese Patent Application Laid-open No. 2000-65832) relates to a filter-form support for biologically specific reaction measurement, the filter-form support enabling pretreatment of a sample to be avoided and correct assay results to be provided, and to a measurement method using the support for biologically specific reaction measurement, and discloses the filter-form support for biologically specific reaction measurement wherein it comprises a filter-form organic polymer having a pore size of 1 to 100 µm. It is reported (p. 4, Column 5, paragraphs [0011] to [0015]) that this filter-form organic polymer can be further subjected, in accordance with the prior art, to at least one type of surface treatment selected from the group consisting of fiber surface hydrolysis, corona discharge, plasma treatment, UV-ozone treatment, and coating with an active material such as a carbodiimide (binder).

It is therefore an object of the present invention to provide an immobilization support that has formed thereon an optimum support surface for immobilizing with specificity, efficiency and good reproducibility various types of substances having different properties, and to provide a solid phase having improved characteristics by use of the support.

### Disclosure of Invention

The immobilization support of the present invention is characterized by the provision of an electrolyte thin film on the surface thereof. That is, the first aspect of the present invention is
(1) an immobilization support wherein an electrolyte thin film is provided on the surface of the support.

With regard to a material of the electrolyte thin film, an inorganic material can be used, but it is preferable to form the electrolyte thin film using a macromolecular material. A second aspect of the present invention is therefore
(2) the immobilization support according to (1) above wherein the electrolyte thin film comprises a macromolecular material.

Furthermore, the electrolyte thin film can be a thin film comprising a polyanion or a polycation, and a third aspect of the present invention is therefore
(3) the immobilization support according to (2) above wherein the electrolyte thin film comprises either a polyanionic thin film or a polycationic thin film.

More preferably, the electrolyte thin film may be formed as a multilayer film by layering a plurality of different polyanionic thin films or polycationic thin films in any combination. Particularly preferably, the electrolyte thin film is formed by alternately layering the polyanionic thin film and the polycationic thin film. That is, a fourth aspect of the present invention is
(4) the immobilization support according to (2) above wherein the electrolyte thin film is formed by alternately layering a polyanionic thin film and a polycationic thin film.

The immobilization support of the present invention can be applied to the fabrication of a solid phase used for, for example, an antigen-antibody reaction, nucleic acid hybridization, a receptor assay, a biosensor, etc. In particular, the immobilization support of the present invention is used for immobilization of, for example, biologically-derived materials, tissue, cells, bacteria, and viruses, or a material that binds thereto or has an affinity therefor, and advantageously provides an optimum solid phase support surface for binding a capturing material to the solid phase support, the capturing material forming a pair with a target measurement substance when capturing the target measurement substance with the solid phase. A fifth aspect of the present invention is therefore
(5) the immobilization support according to any one of (1) to (4) above wherein the immobilization support is used for immobilizing a material that binds to a substance to be detected or a material that has an affinity therefor.

Examples of the target that is immobilized by the immobilization support of the present invention include biologically-derived materials such as proteins, glycoproteins, peptides, glycopeptides, polysaccharides, nucleic acids, lipids, and glycolipids; cells; or a material that binds thereto or a material that has an affinity therefor. A sixth aspect of the present invention is therefore (6) the immobilization support according to any one of (1) to (5) above wherein the immobilization support is used for immobilizing a biologically-derived material such as a protein, a glycoprotein, a peptide, a glycopeptide, a polysaccharide, a nucleic acid, a lipid, or a glycolipid; a cell; or a material that binds thereto or a material that has an affinity therefor.

Furthermore, the present invention also provides a solid phase that employs the immobilization support according to any one of (1) to (4) above and can be applied to various types of measurement and test, the solid phase having immobilized thereon with high specificity, efficiency, and good reproducibility, for example, a material that binds to a substance to be detected or a material that has an affinity therefor, specifically, a biologically-derived material such as a protein, a glycoprotein, a peptide, a glycopeptide, a polysaccharide, a nucleic acid, a lipid, or a glycolipid; a cell; or a material that binds thereto or a material that has an affinity therefor. Seventh and eighth aspects of the present invention are therefore
(7) a solid phase wherein a material that binds to a substance to be detected or a material that has an affinity therefor is immobilized on the immobilization support according to any one of (1) to (4) above, and
(8) a solid phase wherein a biologically-derived material such as a protein, a glycoprotein, a peptide, a glycopeptide, a polysaccharide, a nucleic acid, a lipid, or a glycolipid; a cell; or a material that binds thereto or a material that has an affinity therefor is immobilized on the immobilization support according to any one of (1) to (4) above.

In accordance with each of the above-mentioned constitutions, the present invention provides an immobilization support that enables various types of materials having different properties to be bound with specificity, efficiency, and good reproducibility, and provides a solid phase having excellent characteristics.

### Best Mode for Carrying Out the Invention

The immobilization support of the present invention is provided with an electrolyte thin film on the surface of a substrate of the immobilization support.

With regard to a material of the substrate of the immobilization support of the present invention, any material known to a person skilled in the art may be used, and examples thereof include a plastic such as polystyrene or polycarbonate, a biopolymer material such as cellulose, a glass, a ceramic resin, a metal, and a conductive plastic, but are not limited thereto. Depending on the intended application, these substrates may be in any form such as, for example, microplate, bead, membrane, chromatostrip, chip, tube, needle, or channel.

With regard to the term 'electrolyte thin film' used in the present specification, the term 'electrolyte' is generally used for a water-soluble material, but when forming an electrolyte thin film that can be used in the present invention, it is not always necessary to use a water-soluble material. It is possible to use as a material of the electrolyte thin film of the present invention, for example, an insoluble or oil-based material such as charged microparticles if used as a dispersion in an organic solvent. Moreover, it is not always necessary for the material of the electrolyte thin film of the present invention to be a polymer (macromolecular material); it is also possible to use an inorganic material, etc. other than a macromolecular material, but it is more preferable to use a macromolecular material in terms of ease of forming the thin film and reducing the production cost.

With regard to the macromolecular material, a polyelectrolyte can be suitably used; examples of negative polyelectrolytes include water-soluble anionic polymers such as polyacrylic acid, polymethacrylic acid, polystyrenesulfonic acid, polyparaphenylene (-), polythiophene-3-acetic acid, and polyamide acid, and examples of positive polyelectrolytes include water-soluble cationic polymers such as polyallylamine hydrochloride, polydimethyldiallylammonium chloride, polypyrrole, polyaniline, polyparaphenylene (+), polyparaphenylenevinylene, and polyethylimine, but they should not be construed as being limited thereto.

The electrolyte thin film provided on the substrate surface of the immobilization support of the present invention functions as an adsorbing film that allows various types of materials to bind to the surface of the support with specificity, efficiency, and good reproducibility, and the electrolyte thin film may be formed from one layer of thin film, or may be formed as a multilayer film comprising a plurality of thin film layers. When the electrolyte thin film is formed as a multilayer film, the electrolyte thin film may be formed by layering in any combination a first charged (electrolyte) film having a positive charge and a second charged film having a negative charge. In general, it is preferable to alternately layer the first charged film and the second charged film while taking into consideration repulsion of charges with the same sign. It is also possible to layer a plurality of different types of charged films having a positive charge and a plurality of different types of charged films having a negative charge in any combination or in a manner so that the charges alternate.

When an electrolyte thin film comprising one layer is formed from only one thin film charged either negatively or positively, the surface of the support substrate may, for example, be immersed in either an aqueous solution of the anionic polymer or an aqueous solution of the cationic polymer.

When the electrolyte thin film is formed as a multilayer film, a negative polyelectrolyte thin film and a positive polyelectrolyte thin film are layered in any combination or alternately, and an alternating adsorption multilayer film, which is one type of multilayer (adsorption) film of a negative polyelectrolyte thin film and a positive polyelectrolyte thin film, may be formed using a method described in, for example, G.Decker, Thin Solid Films, 210/211, 831 (1992), D.S.Yoo, Macromolecules, 31, 4309 (1998), and K. Ariga, 'Kagaku to Kogyo' (Chemistry and Industry), Vol. 52, No. 7, pp. 853-856. In general, a procedure of immersing a support in turn in an aqueous negative polyelectrolyte (anion) solution and an aqueous positive polyelectrolyte (cation) solution may be carried out.

Moreover, it is also possible to freely form the electrolyte thin film of the present invention on the support surface by dropping onto, coating, spraying or stamping any section on the support with a polymer solution by means of immersion or by a syringe, a pipette, a dispenser, a brush, an inkjet nozzle, a stamp, or printing. Furthermore, it is also possible to coat a submicroscopic region by means of dip pen nanolithography employing a probe of an atomic force microscope.

The film thickness of the electrolyte thin film to be formed is not particularly limited, but it is preferable that the thickness of one layer is 0.1 to 50 nm, the number of layers is 1 to 1000 layers, and the film thickness is 0.001 to 50 µm; it is more preferable that the thickness of one layer is 0.1 to 50 nm, the number of layers is 1 to 500 layers, and the film thickness is 0.001 to 25 µm, and it is particularly preferable that the thickness of one layer is 0.1 to 50 nm, the number of layers is 1 to 100 layers, and the film thickness is 0.001 to 5 µm. The temperature at which the film is formed is preferably 4°C to 60°C, more preferably 10°C to 45°C, and particularly preferably 15°C to 37°C. Furthermore, the period of time for which immersion in an aqueous anionic polymer solution or an aqueous cationic polymer solution is carried out is not particularly limited, but it is preferably 1 to 60 minutes, more preferably 3 to 30 minutes, and particularly preferably 3 to 15 minutes.

When the alternating multilayer film is formed, after one layer is formed, the next layer may be formed immediately, or the next layer may be formed after a few days have passed. A washing step of the film formation process is typically completed by merely rinsing the support with water. The purity of the water is not particularly restricted as long as it does not interfere with film formation. A liquid used for washing is not necessarily water, and it is also possible to use a solvent in which a macromolecular electrolyte can dissolve.

As hereinbefore described, the term 'electrolyte' is generally used for a water-soluble material, but when the film that can be used in the present invention is formed, it is not always necessary to use a water-soluble material. For example, an insoluble material such as charged microparticles (e.g., ferrite microparticles) or an oil-based material, if it is used in the form of a dispersion in an organic solvent or water, may be used as a material of the electrolyte film of the present invention. It is not always necessary for the material of the electrolyte film of the present invention to be a polymer (macromolecular material). For example, a metal complex monomer can be used as a cationic film, or an inorganic oxide can be used as an anionic film.

The immobilization support having the film formed thereon can be stored at room temperature, and is usually stable for 1 year or longer.

The immobilization support of the present invention enables various types of materials such as, for example, biologically-derived material, tissue, cells, bacteria, and viruses, or a material that binds thereto or has an affinity therefor and, in particular, biologically-derived materials represented by proteins, glycoproteins, peptides, glycopeptides, polysaccharides, nucleic acids, lipids, glycolipids, vitamins, hormones such as steroids, catecholamines, and thyroxine, prostaglandins, various types of neurotransmitter such as GABA, acetylcholine, serotonin, and dopamine, antibiotics, etc.; cells; or a material that binds thereto or a material that has an affinity therefor to subsequently be immobilized thereon, and the immobilization may employ any method known to a person skilled in the art. The solid phase having immobilized on the support thereof various types of materials may be suitably used for various types of antigen-antibody reaction, nucleic acid hybridization, receptor assay, biosensor, etc. employing the solid phase.

As hereinbefore described, in accordance with the present invention, an immobilization support having excellent immobilizing ability and stability can be fabricated by a very simple procedure, and a solid phase having excellent characteristics is provided.

In accordance with the immobilization support of the present invention, by coating the surface of a substrate of the immobilization support with an electrolyte thin film, it becomes possible for a target material to be immobilized on the coated support surface efficiently with good reproducibility, and there are provided an immobilization support having excellent characteristics and a solid phase employing same.

### Examples

The present invention is explained in detail below by way of Examples, but the present invention should not be construed as being limited by the Examples below.

### (Example 1)

A 96-well microplate was immersed in an aqueous polyacrylic acid solution (concentration 10⁻² M) at 25°C for 15 minutes, and then washed with water. During this process, the pH of the aqueous polyacrylic acid solution was maintained at 3.5. Separately, a solution was prepared by adding oligodeoxythymidine (20mer), whose 5'-terminus had been aminated, to a 0.1 M 2-(*N*-morpholino)ethanesulfonic acid (MES) buffer (pH 6), so as to give a concentration of 1.3 mM, and further adding 1-ethyl-3-(3-dimethylaminopropyl)carbodiimide hydrochloride (EDC) thereto to give a concentration of 25 mM. 75 µL of the solution thus prepared was added to each of the wells of the plate, and a reaction was carried out at 60°C for 6 hours so as to immobilize the oligodeoxythymidine on the surface within the wells. Unreacted oligodeoxythymidine was washed away with water, and fluorometry was then carried out by adding 75 µL of a 10 mM tris(hydroxy)aminomethane hydrochloride - 1 mM ethylenediamine-*N,N,N',N'*-tetraacetic acid (TE) buffer (pH 8.0) containing 0.5 vol % of the fluorescence reagent OliGreen. As a separate procedure, the fluorescence reagent was added to a well having no oligodeoxythymidine immobilized thereon, and the fluorescence intensity was measured and deducted as a blank.

When a test was carried out with n = 8, as shown in Table 1, each well of the microplate thus obtained showed a strong fluorescence intensity, suggesting that a large quantity of oligodeoxythymidine was bound.

### (Comparative Example 1)

A 96-well microplate was irradiated with 2000 mJ/cm² ultraviolet rays using a synthetic quartz low pressure mercury lamp. This microplate was used to immobilize oligodeoxythymidine onto the surface within wells in the same manner as in Example 1, and thereafter the procedure of Example 1 was repeated.

When a test was carried out with n = 8, as shown in Table 1, the microplate thus obtained showed a weaker fluorescence intensity compared with that of Example 1, and the reproducibility was poor.

**TABLE 1**

| Well No. | Fluorescence Intensity | |
|---|---|---|
| | Example 1 | Comparative Example 1 |
| 1 | H | L |
| 2 | H | L |
| 3 | H | M |
| 4 | H | L |
| 5 | H | L |
| 6 | H | M |
| 7 | H | M |
| 8 | H | L |
| (Fluorescence Intensity) H:> 5000, M: 2000 to 5000, L: < 2000 | | |

### (Example 2)

A 96-well microplate was immersed in an aqueous polyacrylic acid solution (concentration 10⁻² M) at 25°C for 15 minutes, washed with water, then immersed in an aqueous polyallylamine solution (concentration 10⁻² M) at 25°C for 15 minutes, and washed with water, and these procedures were repeated alternately ten times to form an alternating multilayer film. During this process, the pH of the two aqueous electrolyte solutions was maintained at 3.5. Mite antigen protein (Der f II, manufactured by Seikagaku Corporation) was dissolved in a 100 mM sodium phosphate buffer solution (pH 7.0) to give a concentration of 5 µg/mL, and 100 µL thereof was introduced into the wells of the plate having formed thereon the multilayer film, and was adsorbed at 4°C overnight. The wells were washed with PBS three times, 400 µL of a 1 % bovine serum albumin solution (Diluent/Blocking Solution Concentrate, manufactured by KPL, diluted with distilled water ten times) was added thereto, and a reaction was carried out at 25°C for 1 hour so as to block unreacted solid phase. The wells were washed with PBS three times, 100 µL of a mite-allergic patient serum having mite-specific IgE antibodies was placed in the wells, and a reaction was carried out at 25°C for 2 hours. After the wells were washed with PBS three times, 100 µL of a 1 µg/mL peroxidase-labelled antihuman IgE antibody (manufactured by KPL) was added thereto, and a reaction was carried out at 25°C for 1 hour. The wells were washed with PBS five times, 100 µL of TMB (3,3',5,5'-tetramethylbenzidine, manufactured by KPL) was added thereto and reacted for 3 minutes, and 100 µL of 1 M phosphoric acid was then added to terminate the reaction. The absorbance at 450 nm was measured using a microplate reader. The same procedure was carried out as a negative control for a well with a healthy serum instead of the mite-allergic patient serum. A standard deviation (SD) was determined from fivefold measurement of the negative control, and the MEAN+2SD was determined by doubling this value and adding thereto the mean value of the fivefold measurement. A standard deviation (SD) was also determined from fivefold measurement of the mite allergic patient serum, and the MEAN-2SD was determined by doubling this value and subtracting therefrom the mean value of the fivefold measurement. When the value obtained by subtracting the MEAN+2SD from the MEAN-2SD was 0 or negative, the sample was determined to be negative (-), and when the value was positive, the sample was determined to be positive (+).

The results are given in Table 2. As is clear from Table 2, all of the mite-allergic patients could be determined to be positive (+).

### (Comparative Example 2)

A 96-well microplate was irradiated with 2000 mJ/cm² ultraviolet rays using a synthetic quartz low pressure mercury lamp. This microplate was used to immobilize mite antigen protein in the same manner as in Example 2, and thereafter the procedure and calculation of Example 2 were repeated. As is shown in Table 2, not all of the mite-allergic patients could be determined to be positive (+) from the microplate obtained.

**Table 2**

| Type of serum | | Example 2 | Comparative Example 2 |
|---|---|---|---|
| Mite allergic patient | Serum 1 | Positive (+) | Positive (+) |
| | Serum 2 | Positive (+) | Negative (-) |
| | Serum 3 | Positive (+) | Positive (+) |
| | Serum 4 | Positive (+) | Positive (+) |
| | Serum 5 | Positive (+) | Negative (-) |
| | Serum 6 | Positive (+) | Negative (-) |
| | Serum 7 | Positive (+) | Negative (-) |
| | Serum 8 | Positive (+) | Negative (-) |

### (Example 3)

An aqueous polyallylamine solution (concentration 10⁻² M, pH 5.0) was dispensed into a 96-well microplate, allowed to stand at 25°C for 3 minutes and then washed with water, and an aqueous polyacrylic acid solution (concentration 10⁻² M, pH 2.5) was then dispensed, allowed to stand at 25°C for 3 minutes, and then washed with water. This was repeated alternately 2.5 times to give an alternating multilayer film. Rabbit-derived aldolase was dissolved in a 100 mM sodium phosphate buffer solution (PBS) (pH 7.0) to give a concentration of 1 µg/µL, and 100 µL of this solution was added to the wells of the plate having formed thereon the multilayer film, and was adsorbed at 4°C overnight. The wells were washed with PBS three times, 360 µL of a 1 % bovine serum albumin solution (Diluent/Blocking Solution Concentrate, manufactured by KPL, diluted with distilled water ten times) was then added thereto, and adsorbed at 25°C for 1 hour so as to carry out blocking. The albumin solution was removed, 100 µL of a PBS solution of a peroxidase-labelled aldolase-specific antibody was added to the wells, and a reaction was carried out at 25°C for 2 hours. After the wells were washed with PBS four times, 100 µL of TMB (3,3',5,5'-tetramethylbenzidine, manufactured by KPL) was added thereto and reacted for 15 minutes, and 100 µL of 1 M phosphoric acid was added to terminate the reaction. The absorbance at 450 nm was measured using a microplate reader. When a test was carried out with n = 8, as shown in Table 3, the wells having formed thereon the multilayer film showed a high level of absorbance.

### (Comparative Example 3)

In the same manner as in Example 3, aldolase was adsorbed on a 96-well microplate having no multilayer film formed thereon; after carrying out blocking, a peroxidase-labelled aldolase-specific antibody was added thereto, and the absorbance was measured by a TMB reaction. As shown in Table 3, the absorbance was lower than that of Example 3.

**Table 3**

| Well No. | Example 3 | Comparative Example 3 |
|---|---|---|
| 1 | H | L |
| 2 | H | L |
| 3 | H | M |
| 4 | H | L |
| 5 | H | M |
| 6 | H | L |
| 7 | H | M |
| 8 | H | M |
| (Absorbance) H: > 1.4, M: 1.0-1.4, L: < 1.0 | | |

### (Example 4)

An aqueous polyallylamine solution (concentration 10⁻² M, pH 5.0) was dispensed into a 96-well microplate, allowed to stand at 25°C for 3 minutes, and then washed with water, and an aqueous polyacrylic acid solution (concentration 10⁻² M, pH 2.5) was then dispensed, allowed to stand at 25°C for 3 minutes, and then washed with water. This was repeated alternately 4.5 times to give an alternating multilayer film. Egg white-derived lysozyme was dissolved in a 100 mM sodium phosphate buffer solution (PBS) (pH 7.0) to give a concentration of 10 ng/µL, and 100 µL of this solution was added to the wells of the plate having formed thereon the multilayer film and was adsorbed at 4°C overnight. The wells were washed with PBS three times, 360 µL of a 1 % bovine serum albumin solution (Diluent/Blocking Solution Concentrate, manufactured by KPL, diluted with distilled water ten times) was added thereto, and adsorbed at 25°C for 1 hour so as to carry out blocking. The albumin solution was removed, 100 µL of a PBS solution of peroxidase-labelled lysozyme-specific antibody was added to the wells, and a reaction was carried out at 25°C for 2 hours. After the wells were washed with PBS four times, 100 µL of TMB (3,3',5,5'-tetramethylbenzidine, manufactured by KPL) was added thereto and reacted for 15 minutes, and 100 µL of 1 M phosphoric acid was added to terminate the reaction. The absorbance at 450 nm was measured using a microplate reader. When a test was carried out with n = 8, as shown in Table 4, the wells having formed thereon the multilayer film showed a high level of absorbance.

### (Comparative Example 4)

Lysozyme was adsorbed, in the same manner as in Example 3, on a 96-well microplate having no multilayer film formed thereon; after carrying out blocking, a peroxidase-labelled lysozyme-specific antibody was added thereto, and the absorbance was measured by a TMB reaction. As shown in Table 4, the absorbance was lower than that of Example 4.

**Table 4**

| Well No. | Example 4 | Comparative Example 4 |
|---|---|---|
| 1 | H | L |
| 2 | H | L |
| 3 | H | L |
| 4 | H | L |
| 5 | H | L |
| 6 | H | L |
| 7 | H | M |
| 8 | H | L |
| (Absorbance) H: > 1.2, M: 0.8-1.2, L: < 0.8 | | |

## Claims

1. An immobilization support comprising an electrolyte thin film provided on the surface of the support.

2. The immobilization support according to Claim 1 wherein the electrolyte thin film comprises a macromolecular material.

3. The immobilization support according to Claim 2 wherein the electrolyte thin film comprises either a polyanionic thin film or a polycationic thin film.

4. The immobilization support according to Claim 2 wherein the electrolyte thin film is formed by alternately layering a polyanionic thin film and a polycationic thin film.

5. The immobilization support according to any one of Claims 1 to 4 wherein the immobilization support is used for immobilizing a material that binds to a substance to be detected or a material that has an affinity therefor.

6. The immobilization support according to any one of Claims 1 to 5 wherein the immobilization support is used for immobilizing a biologically-derived material such as a protein, a glycoprotein, a peptide, a glycopeptide, a polysaccharide, a nucleic acid, a lipid, or a glycolipid, a cell, or a material that binds thereto or a material that has an affinity therefor.

7. A solid phase wherein a material that binds to a substance to be detected or a material that has an affinity therefor is immobilized on the immobilization support according to any one of Claims 1 to 4.

8. A solid phase wherein a biologically-derived material such as a protein, a glycoprotein, a peptide, a glycopeptide, a polysaccharide, a nucleic acid, a lipid, or a glycolipid, a cell, or a material that binds thereto or a material that has an affinity therefor is immobilized on the immobilization support according to any one of Claims 1 to 4.
